Europäisches Patentamt

European Patent Office

Office européen des brevets

(11) Publication number: **0 158 647**

**B1**

(12) **EUROPEAN PATENT SPECIFICATION**

(45) Date of publication of patent specification: **02.08.89**

(51) Int. Cl.⁴: **B 65 D 41/00**

(21) Application number: **84903408.7**

(22) Date of filing: **10.09.84**

(86) International application number:
**PCT/US84/01423**

(87) International publication number:
**WO 85/01270 28.03.85 Gazette 85/08**

(54) **PORT PROTECTOR.**

(30) Priority: **16.09.83 US 533076**

(43) Date of publication of application:
**23.10.85 Bulletin 85/43**

(45) Publication of the grant of the patent:
**02.08.89 Bulletin 89/31**

(84) Designated Contracting States:
**BE DE FR GB**

(56) References cited:
**DE-C- 26 894**
**FR-A-2 221 354**
**GB-A- 6 046**
**GB-A- 693 027**
**US-A-3 307 552**
**US-A-4 046 276**
**US-A-4 133 441**
**US-A-4 160 473**
**US-A-4 244 480**
**US-A-4 362 158**
**US-A-4 379 472**
**US-A-4 410 026**

(73) Proprietor: **BAXTER INTERNATIONAL INC. (a
Delaware corporation)
One Baxter Parkway
Deerfield Illinois 60015 (US)**

(72) Inventor: **KARRASCH, Frank
39390 Winchester Road
Wadsworth, IL 60083 (US)**
Inventor: **LOVE, John, F.
848 Carriage Lane
Palatine, IL 60074 (US)**

(74) Representative: **MacGregor, Gordon et al
ERIC POTTER & CLARKSON 14 Oxford Street
Nottingham, NG1 5BP (GB)**

Courier Press, Leamington Spa, England.

## Description

Technical field and prior art

This application relates to an improvement in elastic port protectors which are provided particularly for use in medical equipment to maintain access ports of medical containers of parenteral or peritoneal dialysis solution, for example, or access ports on various sets, in aseptic condition until used.

The Viaflex® collapsible plastic container for parenteral solutions, and the Dianeal® peritoneal dialysis solution container, both sold by Travenol Laboratories, Inc. of Deerfield, Illinois, each carry an injection site made of latex on the end of an injection port, surrounding the end of the access tube and defining inner and outer sleeves in telescoping relation, which respectively pass into the bore of the tube and surround the exterior end thereof. The inner latex sleeve of the port protector is open at its inner end, and connects to a needle pierceable wall at its outer end. The outer sleeve is also connected to the needle pierceable wall at its outer end. A similar structure, having a ring handle, is sold by Abbott Laboratories on peritoneal dialysis solution bags as a port protector.

The outer diameter of the inner sleeve cannot be easily made to exceed the inner diameter of the port in which it resides to improve the seal, since in that circumstance it could be installed into the bore of the port only with great difficulty. The outer sleeve folds around the port to provide a pressure seal. Difficulties also arise in removing the protector from the port.

In Cunningham U.S. Patent No. 4,297,316 and its divisional Patent No. 4,379,472, an end cap port protector is disclosed for thermoplastic tubing which is intended to receive a luer connector, to maintain the dimensional integrity during heat treatment for sterilization or the like. Here also, a latex structure is used having a projecting handle and inner and outer sleeves, the inner sleeve projecting into the bore of the thermoplastic tubing and the outer sleeve surrounding it. The outer end of the cap presents a closed surface to the exterior.

It would be desirable for improved sealing of the port end by the port protector for the inner sleeve to contribute more to the pressure seal formed about the tubular access port by pressure exerted between the outer and inner sleeves or tubular sections against the tubular port. This could be accomplished by causing the inner tubular sleeve to be of larger outer diameter than the inner diameter of the tubular port into which it penetrates. However, previous designs of port protectors would be exceedingly difficult to install into their position on the tubular port in this circumstance, so a compromise has, in the past, been required in which the inner sleeve of the port protector has an outer diameter that is not significantly larger than the inner diameter of the protected tubular port.

DE—C—26 894 discloses an elastic port protector for a soda siphon or the like comprising an inner tubular member plug-engageable in the port and an elastic outer tubular member encircling the inner tubular member in spaced relation thereto, the outer tubular member being sealingly engageable with the outer periphery of the port, the outer tubular member being joined to the inner tubular member at a junction about which the outer tubular member can be folded, the bore of the inner tubular member being closed at the end thereof opposite to the junction and open at the end opposite to the closed end, the inner tubular member being elastic, so that a rod can be inserted in the bore to press against the closed end and stretch the member to permit the latter to be plug-fitted in a port whose inner diameter is smaller than the normal outer diameter of the inner member in its unstretched state, the member being restored to its normal state on removal of the rod so as to engage the port sealingly.

GB—A—693 027 discloses a closure having a peelable outer tubular member but with which difficulties in removal of the closure member also arise, although the member is provided with a tab.

In accordance with this invention, convenient handle means are provided for easy removal of the port protector with relatively low removing force. This is a particular advantage to some patients, for example patients on peritoneal dialysis who may be debilitated and exhibit significant physical weakness so that they have great difficulty in performing normally simple tasks such as removing a port protector from the end of tubing.

The tubular port may be a solution container access port, with the end of the access port occupying the tubular space of the port protector. If desired, the port protector may be positioned on the end of a length of flexible tubing typically at least 20 cm long, with the other end of the flexible tubing communicating with the solution container interior.

The Abbott disclosure, previously referred to, provides an elastic port protector for closing the end of a tubular access port of a medical container of solution and for maintaining the port in aseptic condition until used, the protector comprising an inner tubular member plug-engageable in the port and an elastic outer tubular member (26) encircling the inner tubular member in spaced relation thereto, the outer tubular member being sealingly engageable with the outer periphery of the port, the outer tubular member being joined to the inner tubular member at a junction the port protector additionally comprise a ring-pull handle (40) joined to the outer tubular member at locations spaced from said junction and from each other whereby a pulling force can be applied to the handle to pull the inner tubular member from the port.

The present invention is characterised by the features defined in the characterising part of claim 1.

## Description of the drawings

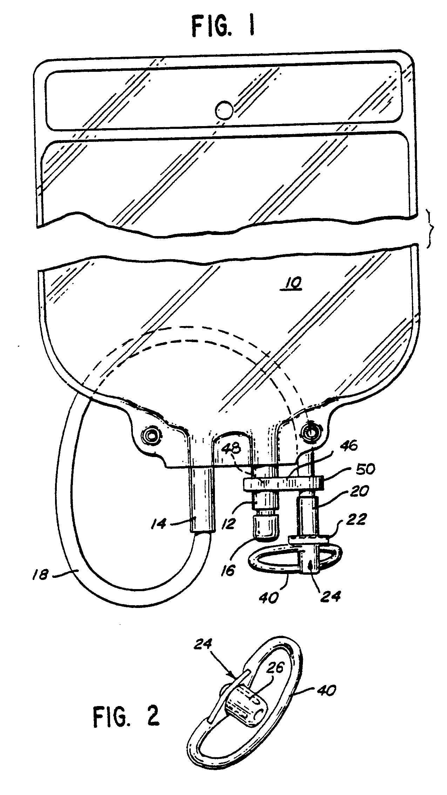

In the drawings, Figure 1 is a fragmentary, plan view of a solution container incorporating the elastic port protector of this invention.

Figure 2 is a perspective view of the port protector of Figure 1.

Figure 3 is a plan view of the port protector of Figure 2.

Figure 4 is a longitudinal sectional view of the port protector of Figure 2, prior to installation on a port.

Figure 5 is a perspective view of the port protector in its everted form, which it normally assumes upon removal from a port.

Figure 6 shows the port protector of the previous drawings being installed on a port.

## Description of specific embodiments

Referring to the drawings, Figure 1 shows a generally conventional solution bag 10 made, for example, of polyvinyl chloride plastic, which may be used as a container for peritoneal dialysis solution. Bag 10 as shown defines a pair of ports 12 and 14, port 12 carrying a conventional latex injection site 16, for example of a type previously described. Port 14 carries a length of tubing 18, preferably in excess of 20 cm, at the end of which is a semirigid tubular connector 20 carrying flange 22 as shown. Port protector 24 fits over the end of tubular connector 20.

Port protector 24 comprises an outer tubular section 26 which defines, relative to associated tubular port 20, an inner end 28 and an outer end 30. Outer tubular section 26 defines an inner diameter in its unstressed condition which is less than the outer diameter of the tubular port 20. For example the unstressed inner diameter of section 26 may be about 0.24 inch (0.61 cm), while the outer diameter of tubular port 20 may be 0.257 inch (0.653 cm). It has been found that no mold draft is required for inner tubular section 32, but a small amount of draft (e.g., 1°) is desirably present for the outer tubular section 32 for ease of molding.

The inner diameter of tubular port 20 may in this circumstance be 0.193 inch (0.49 cm), and the outer diameter of inner tubular section 32 may be about 0.20 inch (0.51 cm).

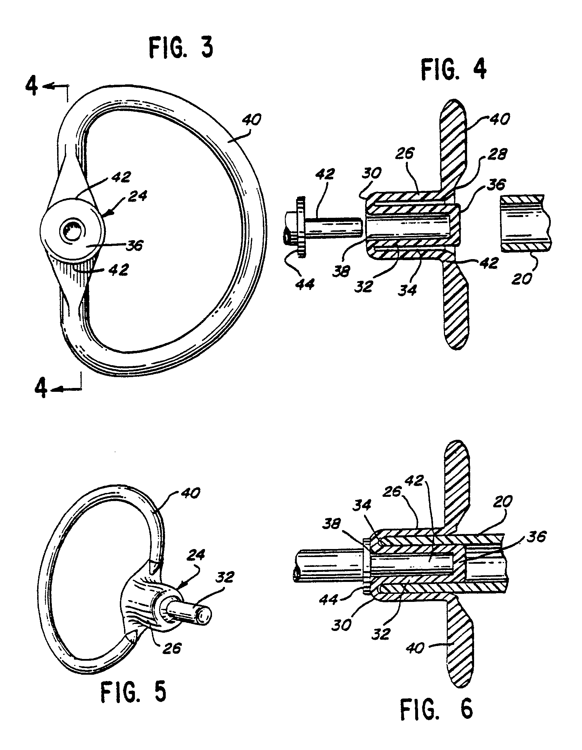

Inner tubular section 32 may be connected to outer tubular section 26 at an annular convolution at end 30 as shown in Figure 4. In other words, inner section 32 represents an integral extension of outer tubular section 26, which is folded or convoluted so as to occupy the bore of tubular section 26, defining a tubular space 34 therebetween.

Inner tubular section 32 defines a closed inner end wall 36 and an open outer end 38.

Handle means 40 is carried on outer tubular section 26, preferably being positioned at the inner end 28 of section 26. Handle means 40 may be a rod of material which is integral with the rest of port protector 24, being connected to outer tubular section 26 at a pair of exactly diametrically opposed positions 42. The effect of this is that when one pulls handle 40 axially, outer tubular section 26 convolutes or folds up in an annular convolution to easily peel off of tubular port 20 for removal, without having to frictionally drag port protector 24 across the surface of tubular port 20, which would greatly increase the pull-off force.

Figure 5 shows port protector 24 in its everted form, following removal by pulling of ring handle 40. Thus, a debilitated person, as many patients who are on chronic peritoneal dialysis are, can be capable of removing the port protector of this invention. At the same time tubular port 20 can be under compressive pressure from both oversized inner tubular section 32 and undersized outer tubular section 26 for improved sealing.

Tubular port 20 may be made of a rigid or semirigid polyvinyl chloride formulation, while port protector 24 may be an integrally molded piece of elastomeric material, for example silicone rubber, or a thermoplastic organic elastomer such as Kraton G, sold by the Shell Chemical Company.

Figure 6 shows how the port protector of this invention may be placed onto tubular port 20 with ease, despite the fact that the inner tubular section 32 may be of a larger outer diameter than the inner diameter of tubular port 20. Because end 38 of inner tubular section 32 is open, one may insert probe member 42 into the bore of inner tubular section 32 so that the end of probe member 42 presses inner end wall 36 into tubular port by a predetermined distance, until flange 44 of probe member 42 engages end 30 of port protector 24. Then probe member 42 may be withdrawn, and outer tubular section 26 may be folded around the exterior of tubular port 20 if necessary for complete installation.

As inner tubular section 32 is advanced by probe member 42, it is slightly stretched, causing it to temporarily reduce its diameter for easier sliding through tubular port 20. Upon removal of probe member 42, inner tubular section 32 retracts back toward its original, unstressed configuration, consequently increasing in diameter, to create the desired internal seal provided by the port protector of this invention.

Thus the port protector of this invention provides improved sealing over previous, related port protectors of the prior art, while at the same time the port protector is easily removed by pulling of the handle, particularly when it is a handle of the type shown, having diametrically opposed connections to outer tubular section 26.

As a further advantage of the port protector of this invention, the fact that the bore of inner tubular section 32 is open to the exterior helps to avoid differential pressure problems during sterilization cycles where there are significant pressure differentials. By this invention the sealed inside area volume around the bore is greatly reduced, which provides advantages in the sterilization process. Additionally, as described in the previously cited Cunningham patents, the tubular port can be protected during heat sterilization from deformation by the presence of inner tubular section 32. Increased protection is provided in this invention because of the greater compressive pressure provided by inner tubular section 32 against tubular port 20 during the heat sterilization processes.

Clip 46 may have an aperture 48 through which port 12 projects to carry clip 46. Tubing 18 can be carried in removable, frictional manner in a slot between jaws 50 for convenient retention of tubing 18 and port 20 until needed for use.

## Claims

1. An elastic port protector (24) for closing the end of a tubular access port (20) of a medical container of solution and for maintaining the port in aseptic condition until used, the protector comprising an inner tubular member (32) plug-engageable in the port and an elastic outer tubular member (26) encircling the inner tubular member in spaced relation thereto, the outer tubular member being sealingly engageable with the outer periphery of the port, the outer tubular member being joined to the inner tubular member at a junction (30) the port protector additionally comprise a ring-pull handle (40) joined to the outer tubular member at locations spaced from said junction (30) and from each other whereby a pulling force can be applied to the handle to pull the inner tubular member (32) from the port, characterised in that the bore of the inner tubular member is closed (36) at the end thereof opposite to the junction (30) and open (38) at the end opposite to the closed end, the inner tubular member (32) being elastic, so that a rod (42) can be inserted in the bore to press against the closed end (36) and stretch the member to permit the latter to be plug-fitted in a port (20) whose inner diameter is smaller than the normal outer diameter of the inner member in its unstretched state, the member being restored to its normal state on removal of the rod so as to engage the port sealingly, and in that the ring-pull handle is joined to the outer tubular member at diametrically opposite portions thereof, whereby a pulling force applied to the handle axially of the tubular members causes the outer tubular member (26) initially to fold up in an annular convolution to peel easily off the port and then to pull the inner tubular member (32) from the port.

2. An elastic port protector according to Claim 1, which is made in one piece of silicone rubber.

3. A container (10) for solution having a length of flexible tubing (18) attached thereto and communicating with the interior thereof, the end of the tubing remote from the container having a port (20) and an elastic port protector according to claim 1 or 2 engaged with the port.

4. A container according to Claim 3 which is a flat-collapsible plastics container carrying a clip (46) for removably retaining the flexible tubing in stowed position on the container.

## Patentansprüche

1. Elastische Öffnungsschutzvorrichtung (24) zum Verschließen des Endes einer rohrförmigen Eingangsöffnung (20) eines medizinischen Lösungsbehälters und zum Erhalten der Öffnung bis zum Gebrauch in aseptischem Zustand, in der die Schutzvorrichtung ein in die Öffnung stöpselartig einsteckbares inneres rohrförmiges Teil (32) und ein das innere rohrförmige Teil in Abstandsbeziehung dazu umschließendes elastische äußeres rohrförmiges Teil (26) aufweist, das äußere rohrförmige Teil in abdichtendem Eingriff mit dem Außenumfang der Öffnung steht, das äußere rohrförmige Teil an das innere rohrförmige Teil an einer Verbindungsstelle (30) angefügt ist, die Öffnungsschutzvorrichtung zusätzlich einen Ringziehgriff (40) enthält, der an das äußere rohrförmige Teil angefügt ist an Stellen, die von der Verbindungsstelle (30) und voneinander beabstandet sind, um das innere rohrförmige Teil (32) aus der Öffnung zi ziehen, dadurch gekennzeichnet, daß die Bohrung des inneren rohrförmigen Teils an dessen der Verbindung (30) entgegengesetzten Ende verschlossen (36) und an dem dem verschlossenen Ende entgegengesetzten Ende offen (38) ist, wobei das innere rohrförmige Teil (32) elastisch ist, so daß ein Stab (42) in die Bohrung eingesetzt werden kann, um gegen das geschlossene Ende (36) zu drücken und um den Teil zu strecken, um dem letzteren zu erlauben, daß er stöpselartig in eine Öffnung (20) eingepaßt wird, deren Innendurchmesser kleiner als der normale Außendurchmesser des inneren Teils in seinem ungestreckten Zustand ist, wobei das Teil beim Entfernen des Stabs in seinen normalen Zustand zurückversetzt wird, um so abdichtend in die Öffnung einzugreifen, und daß der Ringziehgriff an das äußere rohrförmige Teil an diametral entgegengesetzten Abschnitten davon angefügt ist, wodurch eine Ziehkraft an den Griff axial zu dem rohrförmigen Teil angelegbar ist, die das äußere rohrförmige Teil (26) veranlaßt, sich anfänglich in einer ringförmigen Einrollung zusammenzufalten, um sich leicht von der Öffnung abzuschälen, und dann das innere rohrförmige Teil (32) von der Öffnung zu ziehen.

2. Elastische Öffnungsschutzvorrichtung nach Anspruch 1, die einstückig aus Silikongummi hergestellt ist.

3. Lösungsbehälter (10) mit einer an ihm angebrachten und mit seinem Inneren kommunizierenden flexiblen Schlauchabschnitt (18), wobei das von dem Behälter entfernte Ende des Schlauchs eine Öffnung (20) und eine elastische Öffnungsschutzvorrichtung nach Anspruch 1 oder 2, die mit der Öffnung in Eingriff steht, aufweist.

4. Behälter nach Anspruch 3, der ein flach zusammenfaltbarer Kunststoffbehälter ist, der eine Schnelle (46) zur lösbaren Befestigung des flexiblen Schlauchs in eingezogener Stellung am Behälter aufweist.

## Revendications

1. Protecteur élastique d'orifice (24) pour fermer l'extrémité d'un orifice tubulaire (20) d'accès d'un récipient médical de solution et pour maintenir l'orifice en condition aseptique jusqu'à l'utilisation, le protecteur comprenant un élément tubulaire intérieur (32) embrochable dans l'orifice et un élément tubulaire extérieur élastique (26) encerclant l'élément tubulaire intérieur à une certaine distance de celui-ci, l'élément tubulaire extérieur pouvant venir en contact étanche avec la périphérie extérieure de l'orifice, l'élément tubulaire extérieur étant relié à l'élément tubulaire intérieur à l'endroit d'une jonction (30), le protecteur d'orifice comprenant en outre une poignée annulaire de traction (40) reliée à l'élément tubulaire extérieur à des endroits espacés de ladite jonction (30) et l'un de l'autre, de sorte qu'une force de traction peut être appliquée à la poignée pour tirer l'élément tubulaire intérieur (32) hors de l'orifice, caractérisé en ce que le passage de l'élément tubulaire intérieur est fermé (36) à son extrémité opposée à la jonction (30) et ouvert (38) à l'extrémité opposée à l'extrémité fermée, l'élément tubulaire intérieur (32) étant élastique, de sorte qu'une tige (42) peut être insérée dans le passage pour presser contre l'extrémité fermée (36) et étirer l'élément pour permettre à celui-ci de se loger dans un orifice (20) dont le diamètre intérieur est plus petit que le diamètre extérieur normal de l'élément intérieur dans son état non étiré, l'élément reprenant son état normal lors de l'enlèvement de la tige de façon à venir en contact de façon étanche avec l'orifice, et en ce que la poignée annulaire de traction se raccorde à l'élément tubulaire extérieur à des endroits diamétralement opposés de celui-ci, de sorte qu'une force de traction appliquée à la poignée dans la direction axiale des éléments tubulaires provoque le retournement initial de l'élément tubulaire extérieur (26) en un repli annulaire pour décoller facilement cet élément de l'orifice, puis pour tirer l'élément tubulaire intérieur (32) hors de l'orifice.

2. Protecteur élastique d'orifice suivant la revendication 1, qui est fabriqué en une seule pièce de caoutchouc de silicone.

3. Récipient (10) pour solution, comportant une longueur de tube flexible (18) fixée au récipient et communiquant avec son intérieur, l'extrémité du tube éloignée du récipient comportant un orifice (20) et un protecteur élastique d'orifice suivant la revendication 1 ou 2, accouplé à l'orifice.

4. Récipient suivant la revendication 3, qui est un récipient compressible à plat, en matière plastique, comportant une agrafe (46) pour retenir de façon séparable le tube flexible en position arrimée sur le récipient.

FIG. I

FIG. 2

FIG. 3

FIG. 4

FIG. 5

FIG. 6